# EUROPEAN PATENT APPLICATION

(12)

(21) Application number: 84302559.4

(22) Date of filing: 13.04.84

(51) Int. Cl.³: **C 12 N 15/00**, C 12 Q 1/68,
C 12 N 1/20
// (C12N1/20, C12R1/19)

---

(30) Priority: 14.04.83 US 484816

(43) Date of publication of application: 28.11.84
Bulletin 84/48

(84) Designated Contracting States: **AT BE CH DE FR GB IT
LI LU NL SE**

(71) Applicant: **HOWARD HUGHES MEDICAL INSTITUTE,
Coconut Grove Florida (US)**

(72) Inventor: **Woo, Savio L.C., 12806 Palm Desert Lane,
Houston Texas 77099 (US)**
Inventor: **Thirumalachary, T. Chandra c/o T. Venkata
Varadan, 24, Palat Sankaran Road Madhavan Colony,
Numgampakkam Madras-600034 (IN)**
Inventor: **Lidsky, Alan S., 3755 Merrick, Houston
Texas 77025 (US)**
Inventor: **Robson, Kathryn J.H. Nuffield Department of,
Clinical Medicine John Radcliffe Hospital, Headington
Oxford OX3 (GB)**

(74) Representative: **Pennant, Pyers et al, Stevens, Hewlett &
Perkins 5 Quality Court Chancery Lane, London,
WC2A 1HZ (GB)**

---

(54) **Phenylalanine hydroxylase cDNA clones, their methods of production and use in diagnosing classical phenylketonuria.**

(57) Described are phenylalanine hydroxylase cDNA clones, methods of their production, and the use of these clones in the diagnosis of the human genetic disorder, classical phenylketonuria (PKU), a hereditary disorder in phenylalanine metabolism that causes permanent mental retardation in humans, and the identification of heterozygous trait carriers.

EP 0 126 544 A2

-1-

## PHENYLALANINE HYDROXYLASE cDNA CLONES, THEIR METHODS OF PRODUCTION AND USE IN DIAGNOSING CLASSICAL PHENYLKETONURIA

### BACKGROUND OF THE INVENTION

#### Phenylketonuria and its Population Genetics

Phenylketonuria (PKU) is a human genetic disorder caused by an inborn error in aromatic amino acid metabolism. It was first observed some 50 years ago that patients with this condition had excess phenylpyruvate in the urine (Folling, A., 1934a, Nord. Med. Tidskr. 8, 1054-1059; Folling, A., 1934b, Zitschr. Physiol. Chem. 227, 169-176). Subsequently it was discovered that livers of such patients were unable to convert phenylalanine to tyrosine which is the major metabolic pathway of phenylalanine (Jervis, G.A., 1953, Proc. Soc. Exp. Biol. Med. 82, 514-515; Udenfriend, S. and Bessman, S.P., 1953, J. Biol. Chem. 203, 961-966; Wallace et al, 1957, Proc. Soc. Exp. Biol. Med. 94, 63 2-633; Mitoma et al, 1957, Proc. Sco. Exp. Biol. Med. 94, 634-635). Classical PKU is thus characterized by a lack of phenylalanine hydroxylase activity in the liver (Kaufman, S. 1976, In: Advances in Neurochemistry, Vol. 2, pp. 1-132, Agranoff, B.W. and Aprison, M.H., eds., Plenum Press, New York and London). The lack of this enzymatic activity causes persistent hyperphenylalaninemia and minor metabolic pathways for phenylalanine become over-utilized. High levels of phenylalanine and its derivatives are toxic and cause disturbances in tyrosine and tryptophan metabolism

(Blau, K., 1979, In: Aromatic Amino Acid Hydroxylase and Mental Disease (Youdim, M.B.H., ed.) pp. 79-139, New York, Wiley). Diminished formation of catecholamines, malanin and serotonin is typical in untreated PKU patients, and the clinical symptom is an irreversible impairment of brain development resulting in severe mental retardation of the untreated children (Folling, A., 1934a, Nord. Med. Tidskr. 8, 1054-1959; Folling, A., 1934b, Zitschr. Physiol. Chem. 227, 169-176).

Phenylalanine hydroxylase deficiency, regardless of phenotypic variations, is transmitted as an autosomal recessive trait. Massive screening of over 5 million neonates has shown that the prevalence of classical phenylketonuria among Caucasians ranges from 1/5,400 in Ireland to 1/16,600 in Switzerland (Thalhammer et al, 1975, Humangenetik 30, 273-286). The collective frequence in 8 Western European countries and the United States is about 1/8,000 and that 2% of the population are heterozygous carriers of the PKU trait. (Bickel et al, 1973, Acta Paediat. Scand. 62, 413-416; Scriver, C.R. and Rosenberg, L.E., 1973, In: Amino Acid Metabolism and Its Disorders, pp. 290-337, Philadelphia:Saunders, 491 pp.).

Current Methods of Neonatal Screening and Treatment

Phenylketonuric patients secrete large quantities of phenylpyruvate in the urine, which can be readily detected by its reaction with ferric chloride and used in the 1950's as a screening test for diagnosis of PKU children. A simple and mass screening method for a semi-quantitative determination of phenylalanine in small samples of blood from newborn infants was subsequently developed by Guthrie, R. and Susi, A., 1963, Pediatrics 32, 338-343. PKU neonates identified by this test can be treated with a special protein lysate low in phenylalanine content (Bickel et al, 1954, Acta Paediat. Scand. 43, 64-77; Armstrong, M.D. and Tyler, F.H., 1955, J. Clin. Invest. 34, 565-580). This treatment causes a reduced plasma phenylalanine level, disappearance of phenylpyruvate in urine, and is associated with an improvement in mental development and behavioral performance (Koch et al, 1971, In: Phenylketonuria and Some Other Inborn Errors of Metabolism (Bickel, H., Hudson, F.P. and Woolf, L.I., eds.) pp. 20-25; Wamberg, 1977, In: Medico-Social Management of Inherited Metabolic Disease (Raine, D.N., ed.), pp. 63-78, MTP Press Ltd., Lancaster, England; Smith, I. and Wolff, O.H., 1974, Lancet 2, 54 0-544).

Early diagnosis of classical PKU and treatment however, do not achieve uniformly normal IQ scores and learning ability. Initiation of dietary therapy soon after birth has tremendous psychological and social implications for the patient's family (Mikkelson et al, 1974, Narings-forskning 18, 78-86). The dietary correction must be implemented on a prolonged period of time to be effective, and termination of treatment in the mid-first decade is apparently followed by a small but significant decline in IQ scores (Scriver et al, 1980a, N. Eng. J. Med. 303, 1336; Scriver, et al, 1980b, N. Eng. J. Med. 303, 1394). Supervision of this treatment has been shown to be very difficult and can best be performed only at centers experienced with such regimens.

At the present time, there are no prenatal diagnostic methodologies for identification of recessive homozygotes, and the only test available for identification of heterozygotes is the "phenylalanine tolerance" test which is not most quantitative (Woolf et al, 1967, Nature 213, 882-885; Rampini, S., 1973, Schweiz Med. Wschr. 103, 537-546; Guttler, F. and Wamberg, E., 1977, Acta Paeeiat. Scand. 66, 339-344; Guttler, F. and Hansen, G., 1977a, Ann. Clin. Biochem. 14, 124-134; Guttler, F. and Hansen, G., 1977b, Clin. Gen. 11, 137-146). Thus, the development of methodologies for prenatal diagnosis and adult heterozygote detection are desperately needed for prevention, rather than therapy, of this hereditary disorder.

-4-

## The Phenylalanine Hydroxylasing System

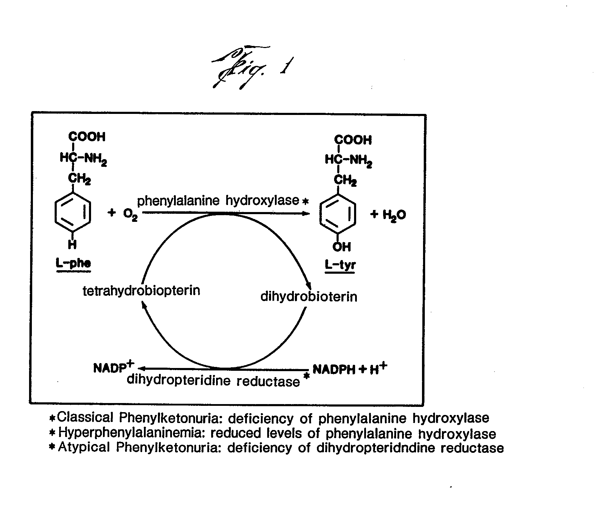

The mammalian phenylalanine hydroxylasing system is complex: it involves several other enzymes and cofactors in addition to phenylalanine hydroxylase itself (Fig. 1). In the presence of molecular oxygen and the active cofactor tetrahydrobiopterin, the enzyme oxidizes phenylalanine to tyrosine and simultaneously converts the cofactor into a quinonoid dihydrobiopterin form (Kaufman, S., 1964, J. Biol. Chem. 248, 540). The quinonoid dihydrobiopterin is subsequently reduced to the tetrahydrobiopterin form by dihydropteridine reductase in the presence of NADPH (Kaufman, S., 1976, In: Advances in Neurochemistry, Vol. 2, pp. 1-132, Agranoff, B.W. and Aprison, M.H., eds., Plenum Press, New York and London). Thus, the pterin cofactor functions catalytically during phenylalanine hydroxylation. Dihydrofolate reductase is required initially to convert the naturally occurring 7, 8-dihydrobiopterin into its active tetrahydro-form (Kaufman, S., 1962, In: Oxygeneses (O. Hayashi, ed.) p. 129, New York, Academic Press). This enzyme however, is no longer a requirement after the initial reduction has occurred because the pterin cofactor recycles only between the tetrahydro-and quinonoid dihydro-forms (Kaufman, S., 1963, Proc. Natl. Acad. Sci. USA 50, 1085). The entire chain of reactions occurs in the liver which is the only tissue to support significant phenylalanine hydroxylating activity in man.

## Heterogenous Phenotypes of Phenylalanine Hydroxylase Deficiency

Since phenylalanine hydroxylation is such a complex system, it is conceivable that hyperphenylalaninemia may also result from deficiency in the other enzymatic activities or cofactors. Indeed variant forms of phenylketonuria have been described recently in which a deficiency in the enzyme dihydropteridine reductase has been demonstrated (Bartholome, K., 1974, Lancet 2:580; Smith et al, 1975, Arch. Dis. Child. 50, 864-870; Kaufman et al, 1975a, New Engl. J. Med. 293, 785-790; Kaufman, et al, 1975b, Pediat. Res. 9, 632-634). The PKU phenotype can also be manifested as the result of dihydrofolate reductase deficiency (Niederwieser, A., 1979, In: Folic Acid in Neurology, Psychiatry and Internal Medicine (Botez, M.I., Reynolds, E.H., eds.) pp. 349-384, New York, Ravin; Danks et al, 1979, Pediatr. Res. 13, 1150-1155) and homeostasis of the tetrahydrobiopterin coenzyme (Danks, D.M., 1978, J. Inherited Met. Dis. 1, 47-48). Nevertheless these types of "atypical phenylketonuria" constitutes a relatively minor proportion of phenylketonuric patients. A vast majority of them still lack phenylalanine hydroxylase enzymatic activity in the liver biopsies, and hydroxylation of phenylalanine does not occur even in the presence of added tetrahydropterin cofactor (Grimm et al, 1975, Clin. Chim. Acta 58, 17-21).

Phenylalanine hydroxylase deficiency however, is a heterogeneous state, since patients with this condition can vary from severe (classical PKU) to moderate (hyperphenylalanemia). Phenylalanine hydroxylase has been purified from livers of rat, monkey and human (Kaufman, S. and Fisher, D.B., 1970, J. Biol. Chem. 245, 4745-4750; Gillam et al, 1974, Biochem. J. 139, 731-739; Choo, K.H. and Cotton, R.G.H., 1979, Biochem. J. 17, 921-946; Woo et al, 1974, Biochem. J. 139, 741-749; Choo et al, 1979a, Biochem. J. 181, 285-294; Choo et al, 1979b, J. Inherited Metab. Dis. 2, 79-84). It is a multimeric enzyme with a subunit molecular weight of about 50,000 daltons. Recent evidence has shown that the subunits are either identical or one being a proteolytic product of the other (Choo et al, 1979a, Biochem. J. 181, 285-294; Choo et al, 1979b, J. Inherited Metab. Dis. 2, 79-84). Phenylalanine hydroxylase from patients with classical phenylketonuria has been shown to be a structurally altered form of the enzyme with less than 1% of the normal activity (Friedman et al, 1973, Proc. Natl. Acad. Sci. USA 70, 552-556), indicating phenylketonuria may be the direct result of a mutation in the phenylalanine hydroxylase gene itself. Furthermore, analysis of

0126544

-6-

phenylalanine hydroxylase activity in liver biopsies of patients with "hyperphenylalaninemia" has shown that they contained about 5% of the normal activity level, with a range of 1 to 35% (Kaufman et al, 1975a, New Engl. J. Med. 293, 785-790; Kaufman, et al, 1975b, Pediat. Res. 9, 632-634; Bartholome et al, 1975, Pediat. Res. 9, 899-903). It has subsequently been demonstrated that the low enzymatic activities in cases of hyperphenylalaninemia was not due to the presence of 5% of the normal enzyme but rather to the presence of an altered hydroxylase with kinetic properties distinct from both the normal enzyme and the enzyme from patients with classical PKU (Kaufman, S., 1976, In: Advances in Neurochemistry, Vol. 2, pp. 1-132, Agranoff, B.W. and Aprison, M.H., eds., Plenum Press, New York and London; Kaufman, S. and Milstein, S., 1977, Ann. Clin. Lab. Sci. 2, 178-185). Together, these studies clearly indicate the presence of multiple phenotypes of phenylalanine hydroxylase deficiency, which could be caused by mutations at various locations in the phenylalanine hydroxylase gene.

SUMMARY

The present invention is directed to phenylalanine hydroxylase cDNA clones, methods of their production, and the use of these clones in the prenatal diagnosis of the human genetic disorder, phenylketonuria (PKU), a hereditary disorder in phenylalanine metabolism that causes permanent mental retardation in humans, and the identification of heterozygous trait carriers.

The mRNA for phenylalanine hydroxylase is purified from total rat liver mRNAs and the purified mRNA is used for synthesis and cloning of its cDNA. The cloned rat phenylalanine hydroxylase cDNA clones are used to identify the corresponding cDNA clones in a human liver cDNA library by cross-hybridization. The human phenylalanine hydroxylase cDNA clones are employed as a hybridization probe to analyze classical phenylketonuria.

Advantageously, standard, approved, recombinant DNA technology is employed and the components, such as the plasmids, etc. are readily available on the market. Details of the invention are set forth below.

BRIEF DESCRIPTION OF THE DRAWINGS

Figure 1 is a schematic representation of the phenylalanine hydroxylation system in rat and man.

Figure 2 is a Coomassie blue-stained polyacrylamide gel containing various rat phenylalanine hydroxylase fractions throughout the purification procedure.

Figure 3 is an Ouchterlony analysis of goat anti-rat phenylalanine hydroxylase antiserum.

Figure 4 is a schematic representation of the immunoprecipitation procedure employed for purification of phenylalanine hydroxylase mRNA from rat liver.

Figure 5 is a schematic representation of the cell-free translation procedure employed to detect the activity of rat liver phenylalanine hydroxylase mRNA.

Figure 6 are fluorograms of polyacrylamide gels containing cell-free translation products directed by total rat liver polysomal mRNA and purified rat liver phenylalanine hydroxylase mRNA. Panel A, total translation product; Panel B, immunoprecipitated translation product using goat anti-rat liver phenylalanine hydroxylase antiserum.

Figure 7 are radioautograms of agarose gels containing DNAs isolated from various rat liver cDNA clones after hybridization with $^{32}$P-labelled cDNA synthesized from polysome-enriched phenylalanine hydroxylase mRNA [Panel A] and polysome-depleted mRNA [Panel B].

Figure 8 is a schematic representation of hydrid-selected translation procedure for confirmation of the identity of rat phenylalanine hydroxylase cDNA clones.

Figure 9 are fluorograms of polyacrylamide gels containing cell-free translation products directed by mRNA selected by various plasmid DNA. Panel A, total cell-free translation products; Panel B, immunoprecipitated cell-free translation products using goat anti-rat phenylalanine hydroxylase antiserum.

Figure 10 is a radioautogram of an agarose gel containing various mRNA preparations after hybridization with $^{32}$P-labelled prPH98 DNA.

Figure 11 is a schematic representation of simplified restriction maps of two rat phenylalanine hydroxylase cDNA clones with largest overlapping inserted DNA fragments.

Figure 12 is a radioautogram of an agarose gel containing DNA isolated from two rat and six human phenylalanine hydroxylase cDNA clones after hybridization with $^{32}$P-labelled rat phenylalanine hydroxylase DNA isolated from prPH98 DNA.

Figure 13 is a schematic representation of two human phenylalanine hydroxylase cDNA clones with largest overlapping inserted human DNA fragments.

Figure 14 are radioautograms of agarose gels containing genomic DNA isolated from normal caucasians after digestion with various restriction enzymes and hydridization with $^{32}$P-labelled phPH72 and phPH73 DNAs. Panel A, MspI; Panel B, SphI; and Panel C, HindIII.

Figure 15 are radioautograms of agarose gels containing genomic DNAs isolated from member of three PKU families after hybridization with $^{32}$P-labelled phPH72 and phPH73 DNAs. The restriction enzymes used in Panel A, B, and C are MspI, SphI and HindIII, respectively. Schematic representation of Mandelian segregation of the PKU genes in these families are illustrated on the right of the corresponding radioautograms.

Figure 16 are radioautograms of agarose gels containing genomic DNAs isolated from members of two additional PKU families after digestion with HindIII and hybridization with $^{32}$P-labelled phPH72 and phPH73 DNAs. The Mandelian segregation of the PKU genes in these families are shown schematically with the corresponding radioautograms.

Figure 17 is a radioautogram of an agarose gel containing genomic DNAs isolated from members of a sixth PKU family after digestion with either HindIII or SphI and

hybridization with $^{32}$P-labelled phPH72 and phPH73 DNAs [Panel A]. The Mandelian segregation of the PKU genes in this family is shown schematically in Panel B.

PRESENTLY PREFERRED EMBODIMENTS

Molecular and Biological Basis of Classical Phenylketonuria

The following organisms are available from permanent collection of the American Type Culture Collection, 12301 Parklawn Drive, Rockville, Maryland 20852, U.S.A.

| | |
|---|---|
| ATCC 39329 | E. Coli RRl (prPH91) |
| ATCC 39330 | E. Coli RRl (prPH98) |
| ATCC 39331 | E. Coli RRl (phPH72) |
| ATCC 39332 | E. Coli RRl (phPH73) |

The deposits are available to the public upon a grant of a patent to the Assignee, Howard Hughes Medical Institute, disclosing them. It should be understood, however, that the availability of a deposit does not constitute a license to practice the subject invention in derogation of patent rights granted by governmental action.

By recombinant DNA technology, the genes coding for the normal and variant proteins are cloned and their nucleotide sequences determined. Comparison of the amino acid sequences of the entire proteins deduced from the gene sequences reveals any amino acid substitutions and thereby establishes the molecular basis of the deficiency at the gene level. Within the phenylketonuric phenotype, there could exist a number of genotypic alleles due to genomic polymorphism in man. These genotypic alleles could be the result of nonsence mutations, conservative amino acid substitutions, classical silent mutations, intervening sequence divergence and flanking sequence polymorphism, which may not be manifested at the protein level. The existence of immunoreactive materials were present and absent in liver biopsies of patients with classical PKU (Choo, et al, 1979a, Biochem. J. 181, 285-294; Choo, et al, 1979b, J. Inherited Metab. Dis. 2, 79-84; Bartholome, K. and Ertel, 1976, Lancet 2:862-863; Bartholome, K. and Ertel, 1978, Lancet 1:454).

Adult Heterozygote Detection and Prenatal
Diagnosis of Classical PKU

Having established the biochemical basis of the
genetic lesions for classical PKU, the identification of
polymorphic nucleotides between the normal and the variant
genes giving rise to the appearance and disappearance of
certain restriction endonuclease cleavage sites, which may
or may not be located at the sites of the genetic lesions
are made. This body of information is then catalogued so
that the differences in restriction endonuclease cleavage
patterns of genomic DNA fragments containing phenylalanine
hydroxylase gene sequences can be used in combination with
amniocentesis as a diagnostic tool to identify prenatally
the recessive homozygotes who are predisposed to development
of severe mental retardation.

Most importantly, the same analytical method can
be used to identify the adult heterozygous carriers. It has
recently been shown that heterozygosity may also impose a
risk on the patient's health state (Ford, R.C. and Berman,
J.L., 1977; Lancet 1, 767-770; Thalhammer et al, 1975,
Humangenetik 30, 273-286; Bessman et al, 1978, Proc. Natl.
Acad. Sci. USA 75, 1562-1566). Early identification of
these individuals will be necessary for prevention of this
genetic disorder.

In order to isolate the human chromosomal phenylalanine hydroxylase gene, an appropriate hybridization probe
must first be available. Although the best probe is a cDNA
clone of human phenylalanine hydroxylase mRNA, the latter is
not readily available since the supply of fresh human tissue
needed for isolating intact mRNAs cannot be relied upon. We
have elected to use rat liver (although other animal liver
tissue can be used) for mRNA isolation and for construction
of the corresponding cDNA clones, which are then used to
identify clones containing the corresponding human cDNA and
chromosomal gene from human liver cDNA and genomic DNA
libraries by cross hybridization.

Enrichment of Phenylalanine Hydroxylase Messenger
RNA by Polysome Immunoprecipitation

Most eukaryotic messenger RNAs are not present at high enough concentrations in the cell for direct cloning, and the best available procedure to enrich these mRNAs is by immunoprecipitating specifically the polysomes engaged in the synthesis of the corresponding proteins (Palacois et al, 1973, J. Biol. Chem. 248, 540). Phenylalanine hydroxylase was thus purified for the generation of mono-specific antibodies.

Purification of Phenylalanine Hydroxylase From Rat Liver

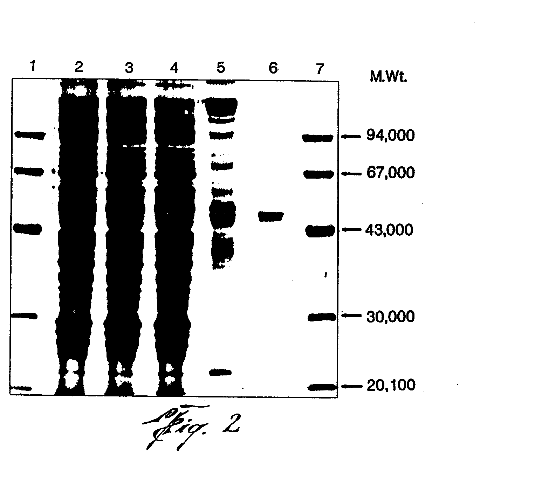

Purification of phenylalanine hydroxylase from rat liver was carried out by a modification of the procedure of Shiman et al, 1979, J. Biol. Chem. 254, 11306. Protein purity was monitored by Coomassie blue-staining of 10% polyacrylamide slab gels (Fig. 2). Briefly the proteins in total rat liver homogenate were clarified by high speed centrifugation. Phenylalanine hydroxylase was activated by incubation at 25°C for 30 minutes prior to fractionation by phenyl-sepharose column chromatography. The eluate was further purified by a DEAE-cellulose column. The resulting enzyme preparation contained 2 bands at 50,000 daltons as previously reported (Fisher, D.B. and Kaufman, S., 1973, J. Biol. Chem. 248, 4345-4353; Gillam et al, 1974, Biochem. J. 139, 731-739), and appeared to be greated than 95% pure with an overall purification of about 400-fold (Fig. 2, lane 6).

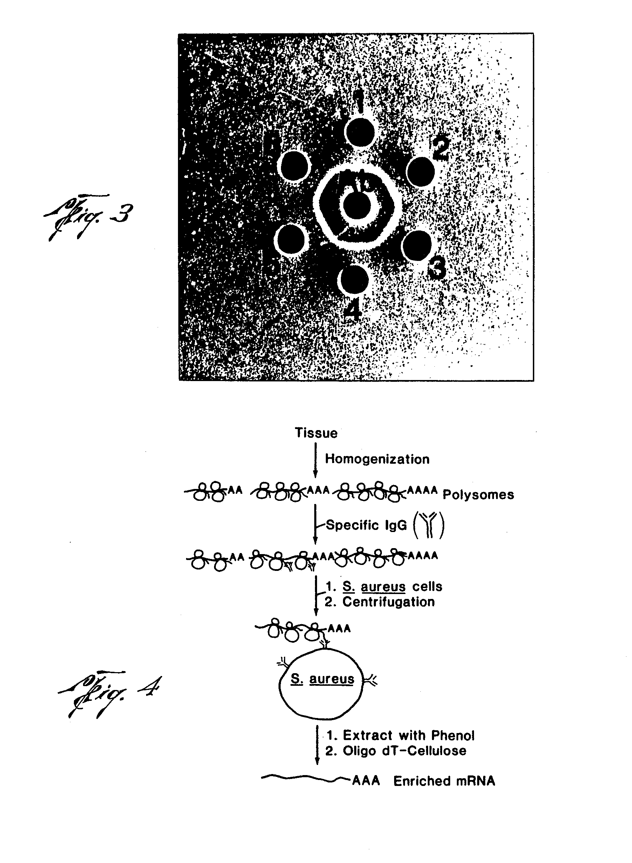

Antibody against rat liver phenylalanine hydroxylase was raised in a goat and analysed by double immuno-diffusion on an Ouchterlony plate. The center well contained crude antiserum, with increasing quantities of crude rat liver homogenate and purified phenylalanine hydroxylase in the periphery wells. One continuous precipitation band was observed, indicating that the goat antibody to rat liver phenylalanine hydroxylase is monospecific (Fig. 3). The IgG fraction of the anti-serum was also capable of inhibiting phenylalanine hydroxylase activity in a dose-dependent manner.

Purification of Ribonuclease-free
Antibody to Phenylalanine Hydroxylase

Antibody to rat liver phenylalanine hydroxylase was purified from crude anti-serum by affinity chromatography. Purified antigen was coupled to cyanogen bromide-activated sepharose. From then on all reagents and procedures were carried out at 4°C. The antiserum was applied to the affinity resin and was washed thoroughly with PBS (10mM sodium phosphate, pH 7.4, containing 0.15 M NaCl). Any remaining non-specific protein and trace amounts of contaminating ribonuclease were further removed by washing the column with 3 volumes of 0.2 M KCNS in PBS as outlined by Gough, N.M. and Adams, J.M., 1978, Biochemistry, 17, 5560. The specific antibody was eluted from the affinity resin by the addition of 0.5 M acetic acid, and was neutralized immediately by the addition of ammonium hydroxide. The purified antibody was dialyzed extensively against PBS before use. The antibody was tested for ribonuclease activity by incubating the antibody with polysomes under the same conditions to be used for immunoprecipitation. The profiles of the polysomes before and after the antibody treatment were then compared by sucrose density gradient centrifugation.

Isolation of Rat Liver Polysomes

Rat liver polysomes were isolated using the magnesium precipitation procedures as described by Palmiter (1974, Biochemistry, 13, 3606) and modified by Lee et al, (1978, J. Biol. Che., 253, 3494) as outlined below. The frozen liver was pulverized and a 10% liver homogenate was prepared in a cold buffer containing 2% triton X-100 and 1 mg/ml of heparin using a teflon homogenizer. The homogenate was immediately centrifuged at 10,000 rpm for 30 seconds and the supernatant containing both bound and free polysomes were precipitated by adjusting the magnesium concentration to 100 mM. The polysomes were allowed to aggregate for one hour on ice and the precipitated polysomes were separated from the remaining cellular matter by centrifuging through 0.5 M sucrose pads in the presence of 0.5% triton X-100 and 0.5 mg/ml of heparin. The resulting polysomes were dissolved in 10 mM Hepes, pH 7.5, containing 5 mM $MgCl_2$, 0.15 M NaCl and 0.5% triton X-100 with the aid of a glass teflon homogenizer. The particulate matter was removed by centrifugation and the clarified supernatant containing the polysomes usually has an A260/A280 ratio of 1.5 to 1.8. The yield has generally been 50 $A_{260}$ units of polysome per gram of liver. The polysome sedimentation profiles were checked for any signs of RNA degradation by sucrose density gradient centrifugation, and the majority of the polysomes prepared in this manner contained more than 10 ribosome units.

Immunoprecipitation of Phenylalanine Hydroxylase-Synthesizing Polysomes

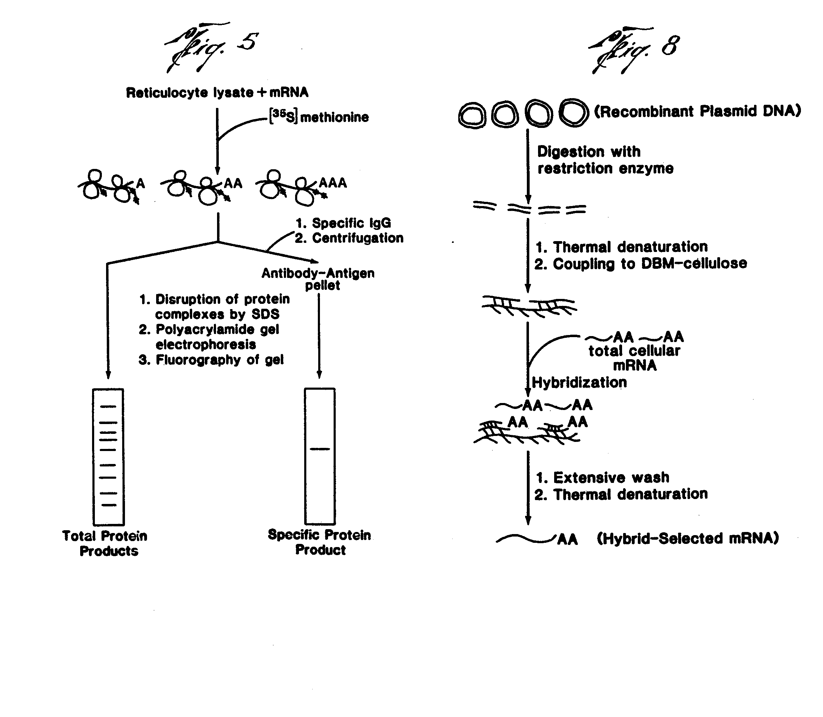

The procedure used was that described by Gough and Adams (1978, Biochemistry, 17, 5560) and as illustrated in Fig. 4. The rat liver polysomes (2500 A260 units) were incubated with 3 mg of ribonuclease-free antibody at 4°C in the presence of 100 µg/ml of heparin. The resulting antibody-polysome complexes were incubated with S. aureus cells in the presence of 2 mM EGTA, which is a potent inhibitor of calcium-dependent nucleases. The cells had been washed extensively with three different washing buffers containing 0.5% deoxycholate and 2 mM EGTA prior to use. The bacteria-IgG-polysome complexes were sedimented through discontinuous sucrose gradients. The pellets were resuspended in a buffer containing 5 mM $MgCl_2$ and 2 mM EGTA, and sedimented through sucrose for a second time to remove residual non-specific polysomes. RNA was then released from the bound polysomes with a buffer containing SDS/EDTA and the bacteria were removed from centrifugation. The resulting supernatant was deproteinized by extraction with phenol/chloroform, and the RNA was precipitated with ethanol at -20°C. Poly(A)-containing RNA was isolated by oligo(dT)-cellulose column chromatography. An intermediate 0.2 M KCl wash was included prior to the elution of the poly(A)-containing RNA with water.

0126544

-14-

Assessment of Purity of Polysome-Enriched
Phenylalanine Hydroxylase mRNA

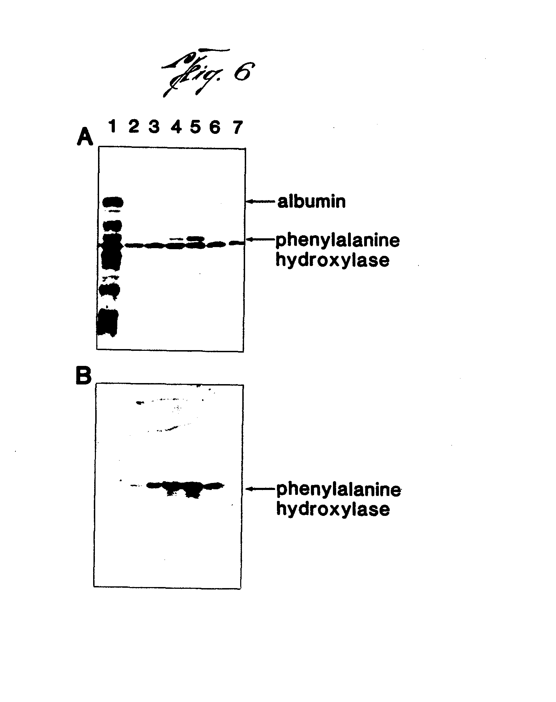

Poly(A)-containing RNA fractions eluted from the oligo (dT) cellulose column were assayed using the mRNA-dependent rabbit reticulocyte cell-free translation system (Pelham and Jackson, 1976), using [$^{35}$S] methionine as the radioactive tracer. Aliquots from the translation mixtures were denatured and subjected to polyacrylamide gel electrophoresis according to the procedure of Laemnli (1970, Nature, 227, 680). Gels were prepared for fluorography using NEN enhancer and dried on to Whatman 3MM paper before exposure to Kodak X-ray films. The remainder of the cell-free translation mixture was immunoprecipitated and the products were also subjected to electrophoresis and fluorography (Fig. 5).

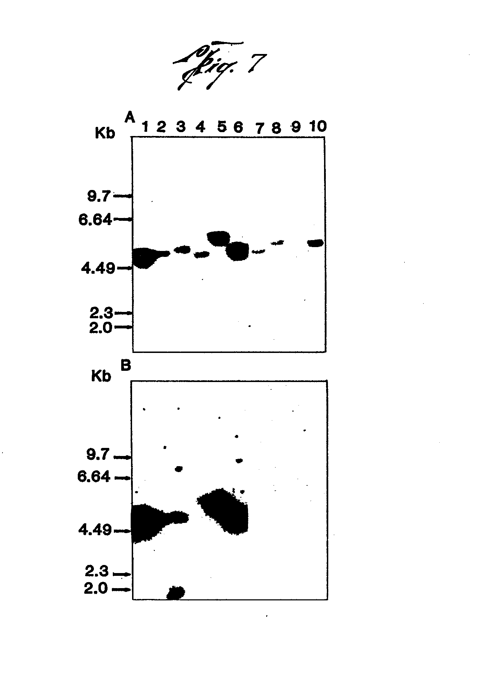

Phenylalanine hydroxylase constitutes about 0.25% of total liver proteins and its mRNA is expected to be present at correspondingly low levels. When cell-free translation products directed by total rat liver polysomal RNA were analysed by SDS-gel electrophoresis and fluorography, multiple protein bands were observed with the most prominent one being the 69,000 dalton albumin (Fig. 6A, lane 1). Only a minor portion of the protein products was immunoprecipitated with anti-phenylalanine hydroxylase as expected (Fig. 6B, lane 1). On the other hand, it is apparent that a major protein product synthesized in response to the polysome-enriched RNA preparation has a molecular weight of about 50,000 (Fig. 6A, lanes 3-6). This protein product was quantitatively precipitated with anti-phenylalanine hydroxylase (Fig. 6B, lanes 3-6). The polysome-enriched phenylalanine hydroxylase mRNA preparation appears to be in excess of 20% in purity, and a minimum of 80-fold enrichment from total rat liver mRNA was achieved in a single step.

Construction of Recombinant cDNA Clones
From Polysome-Enriched Phenylalanine Hydroxylase mRNA Enzymatic Synthesis of Complementary DNA

Five µg of the highly purified phenylalanine hydroxylase mRNA was incubated in a final volume of 200 μl at 46° for 30 minutes with 80 units of AMV reverse transcriptase in the presence of 50 mM Tris-HCl, pH 8.3, 50 mM KCl, 10 mM $MgCl_2$, 40 mM 2-mercaptoethanol, 12.5 μg/ml of oligo (dT), 40 μg/ml Actinomycin D, and 1 mM each of the 4 deoxyribonucleoside triphosphates. 100 μCi of [$^3$H]dCTP was used as radioactive tracer. The reaction was stopped by the addition of EDTA to 20 mM, and the mixture was de-proteinized by extraction with phenol. The aqueous phase was chromatographed through a small Sephadex G-25 column. The nucleic acid eluted in the void volume was adjusted to contain 0.1 N NaOH and 10 mM EDTA. The RNA was hydrolysed by incubating the mixture at 68° for 30'. After neutralizing the mixture with HCl, synthetic DNA was precipitated from ethanol. From the radioactive dCMP incorporated, it was estimated that about 1 μg of cDNA was synthesized. The single stranded cDNA was then purified by centrifugation on an 8-18% linear sucrose gradient in the presence of 0.1 M NaOH. The radient was developed by centrifugation at 38,000 rpm for 24 hours in a SW40 rotor at 4°C. The fractions containing DNA greater than 1300 nucleotides were pooled, neutralized with 2M NaOAc, pH 5.0 and precipitated with ethanol.

Enzymatic Synthesis of Double Stranded DNA

Single stranded cDNA contains a terminal hairpin loop at the end which can serve as a template as well as a primer for double stranded DNA synthesis. The single stranded [$^3$H]cDNA preparation was thus made double stranded by incubating at 46° for 90 minutes in a final volume of 100 μl with 400 units/ml of reverse transcriptase in 50 mM Tris-HCl, pH 8.3, 10 mM $MgCl_2$, 40 mM 2-mercaptoethanol and 1 mM each of the 4 deoxyribonucleoside triphosphates. This time 25 μCi of [$^{32}$P]dCTP was used as tracer. The reaction was again stopped by addition of EDTA and deproteinized by phenol extraction. The mixture was chromatographed through a small Sephadex G-25 column and nucleic acid eluted in the void volume pooled. A total 0.4 μg of double stranded DNA was obtained by this procedure.

-16-

S1 Nuclease Treatment of Double Stranded DNA

The double stranded DNA synthesized in the above manner contains a terminal loop which can be opened up by treatment with S1 nuclease which specifically digest single stranded DNA. The double stranded DNA (2 µg/ml) was incubated at room temperature for 1 hour with 4500 units/ml of S1 nuclease in 0.03 M NaOAc, pH 4.5, 0.3 M NaCl and 4.5 mM $ZnSO_4$. The reaction was stopped with EDTA and S1 nuclease was inactivated by phenol extraction. The aqueous phase was chromatographed through a small Sephadex G-25 column, and 0.25 µg of double stranded DNA was eluted at the void volume.

Tailing double stranded DNA with dCTP using terminal transferase

The S1-treated double stranded DNA was incubated with 200 units of calf thymus deozynucleotidyl terminal transferase in a final volume of 100 µl at 37° for 5 minues in the presence of 5 µM [$^3$H]dCTP, 0.2 M potassium cacodylate, pH 7.2, 1 mM $CoCl_2$ and 1 mM mercaptoethanol. Approximately 15 dCMPs were added per DNA terminus. The reaction was again stopped with EDTA and the enzyme was inactivated by heating at 68° for 10 minutes. The mixture was chromatographed through a small Sephadex G-25 column.

Reannealing with dG-tailed pBR322 and Transformation of Bacteria

The plasmid vector pBR322 was linearized by digestion with Pst I and 14 dGMP's were added per DNA terminus using terminal transferase. Equimolar quantities of dC-tailed synthetic DNA and dG-tailed pBR322 were mixed in reannealing buffer (10 mM Tris-HCl, pH 7.8, 0.1 M NaCl). The final plasmid DNA concentrations was 2 µg/ml. The mixture was heated at 68° for 30 minutes, followed by incubation at 43° for 3 hours. The mixture was then allowed to slow cool at room temperature (about 3 hours) and left at room temperature overnight. The mixture was then employed for transformation of E.. coli RRI according to previously published procedures.

Identification of bacterial colonies carrying recombinant
plasmids

Individual bacterial transformants were hand-picked onto fresh agar plate in a gridded pattern and allowed to grow to sufficient sizes. The colonies were then lifted onto a nitrocellulose filter which was allowed to flow for 30 minutes on a Whatman 3 MM paper soaked with 0.5 N NaOH, 1.5 NaCl. During this period, the bacteria were lysed, their DNAs denatured and become bound to the nitrocellulose. The filters were then neutralized, baked and allowed to hybridize with $[^{32}P]cDNA$ synthesized against 0.5 µg of the purified phenylalanine hydroxylase mRNA preparation. After extensive washing, the bacterial colonies containing recombinant plasmid DNAs were identified by radioautography.

Identification of phenylalanine hydroxylase cDNA clones

In the absence of a published amino acid sequence for rat liver phenylalanine hydroxylase, the identity of the phenylalanine hydroxylase cDNA clones cannot be established by DNA sequencing. The fidelity of these clones was provided by 3 independent lines of evidence: a. differential hybridization; b. hybrid-selected translation and c. Northern blot analysis.

Selection of Candidate Phenylalanine Hydroxylase cDNA
Clones by Differential Hybridization

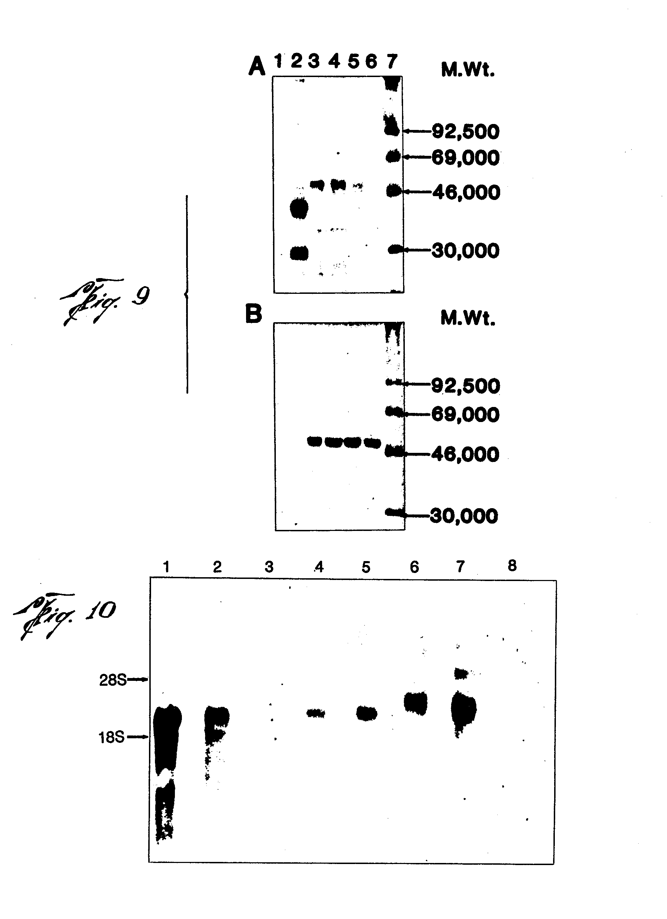

Recombinant plasmid DNAs were prepared by the mini-lysate procedure of Birnboim and Doly (1979, Nucleic Acid Research, Vol. 1, pp. 1513-1523), digested with Bam HI and resolved by agarose gel electrophoresis. The DNA was transferred bi-directionally to two nitrocellulose filters according to the procedure of Smith and Summers (1980, Analytical Biochemistry, Vol. 109, pp. 123-129). The identical filters were hybridized separately to two $[^{32}P]cDNA$ probes: Fig. 7A and 7B show the radioautograms of the filters after hybridization with $[^{32}P]cDNAs$ synthesized from polysome-enriched mRNA and depleted mRNA preparations, respectively. Differential hybridization signals were observed with several clones (lanes 4, 7, 8 and 10), and
they represent candidates to contain phenylalanine hydroxylase DNA sequences. The number of colonies positive to
this differential hybridization test represent about 20% of
the total number of recombinants. This number correlates
well with the estimated purity of phenylalanine hydroxylase
mRNA used for cloning.

Confirmation of Phenylalanine Hydroxylase Clones by
Hybrid-Selected Translation

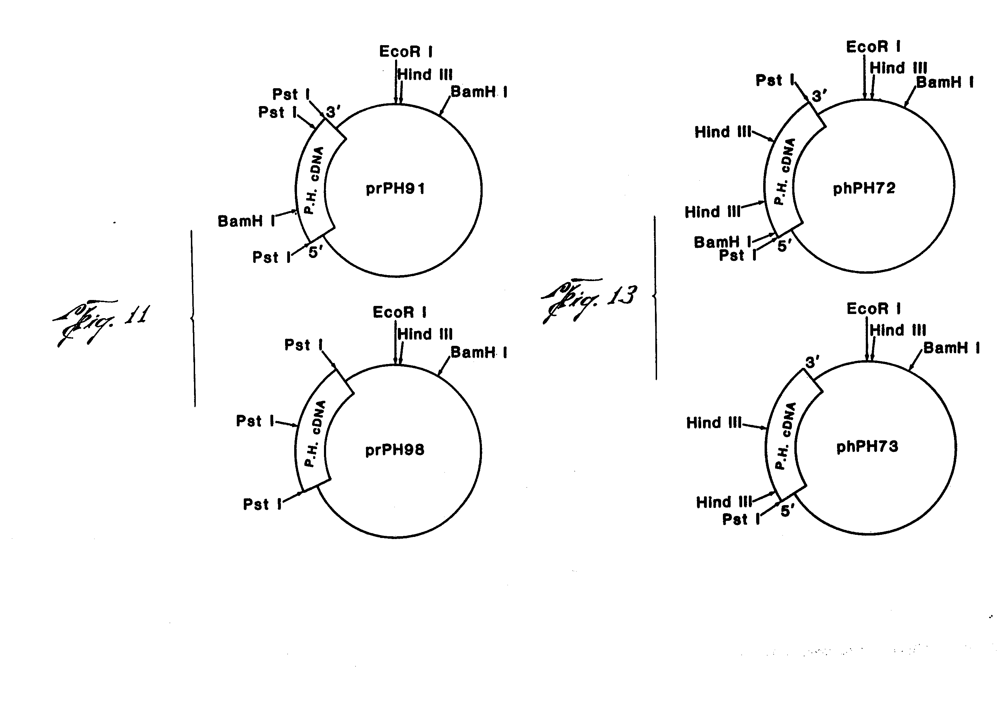

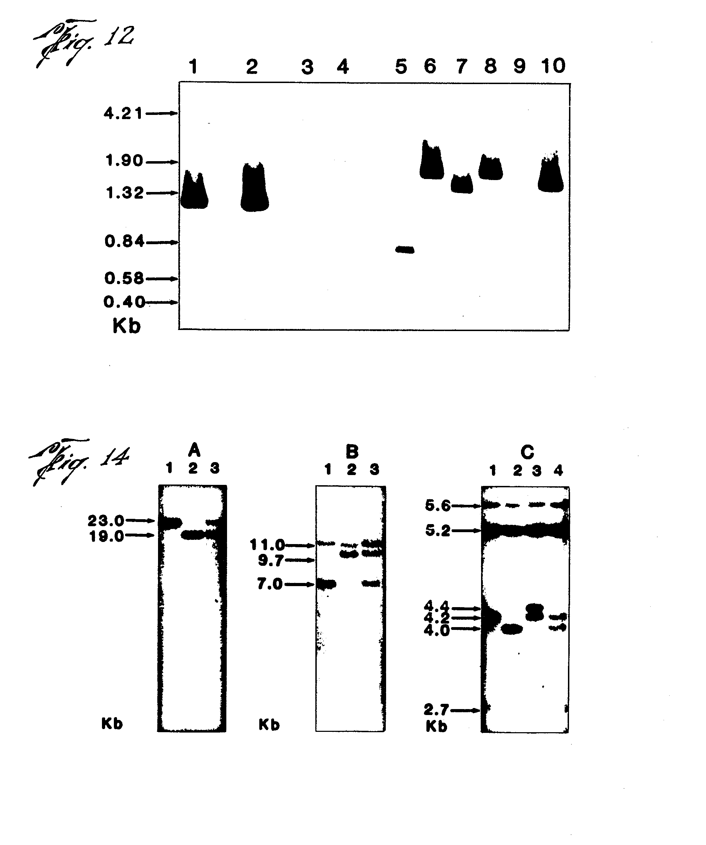

Candidate recombinant clones were further analyzed
by hybrid-selected translation (Fig. 8). Plasmid DNA
isolated from these colonies were denatured and coupled to
aminobenzyloxymethylcellulose. The immobilized DNA was
allowed to hybridize with total rat liver poly(A)-containing
RNA. RNA eluted from the resins were translated in vitro
and total protein products were analyzed by electrophoresis
on SDS-polyacrylamide gels followed by fluorography. In
this experiment, the 50,000 dalton band endogenuous to the
recticylocyte lysate system was selectively removed by
boiling the gel in 10% trichloroacetic acid. Products
syntehsized in response to mRNA selected by the cloning
vector pBR322 DNA as the negative control are shown in Fig.
9A, lane 1. The profile is similar to that of total rat
liver poly(A)-containing RNA and very little, if any, of the
product is immunoprecipitable with the specific antibody
against phenylalanine hydroxylase (Fig. 9B, lane 1). As
positive controls for hybrid selection, two independent rat
cDNA clones coding for 2 smaller molecular weight liver
proteins were included in the test, and two intense bands
were obtained (Fig. 9A, lane 2), with no immunoprecipitable
phenylalanine hydroxylase as expected (Fig. 9B, lane 2).
Protein products synthesized in response to mRNAs selected
by the candidate clones however, contained a major band at
50,000 daltons, suggesting the presence of phenylalanine
hydroxylase (Fig. 9A, lanes 3-6). That these protein bands
were precipitated quantitatively with anti-phenylalanine hydroxylase (Fig. 9B, lanes 3-6) confirmed that these clones do contain phenylalanine hydroxylase DNA sequences.

RNA Analysis by Northern Hybridization

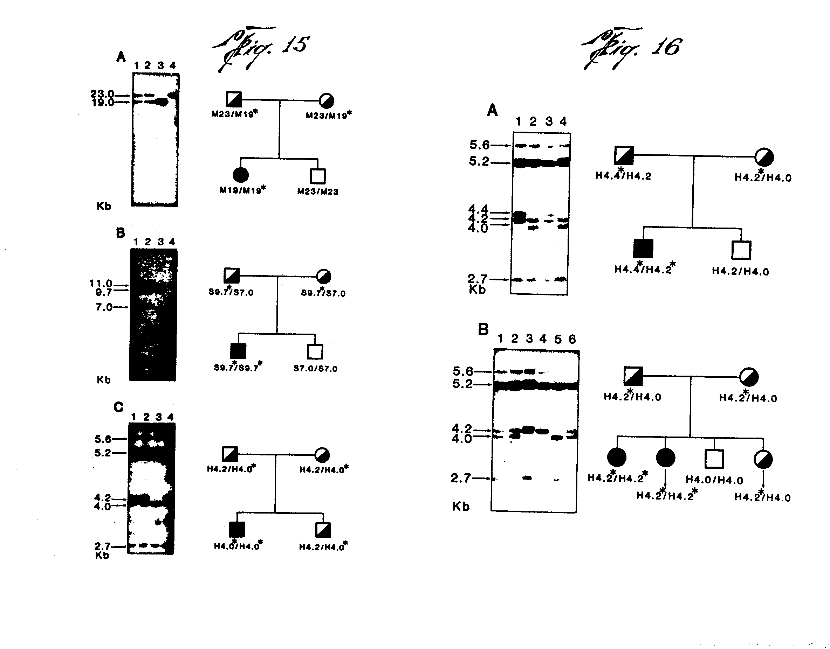

Total poly(A)-containing RNA was extracted from various rat and primate tissues and their phenylalanine hydroxylase mRNA contents were analyzed using a rat liver phenylalanine hydroxylase cDNA clone (prPH98) as the hybridization probe (Fig. 10). A hybridization signal of about 21S was obtained for total rat liver poly(A)-containing RNA (lane 2). The fact that 0.5 μg of the polysome-enriched phenylalanine hydroxylase mRNA preparation generated a much more intense signal (lane 1) than 5 μg of total rat liver poly(A)-containing RNA provided further support for the fidelity of the cDNA clone. No hybridization signal was obtained with RNA from rat heart (lane 3), indicating the tissue specificity of the cDNA probe. The rat kidney is known to contain phenylalanine hydroxylase activity and a corresponding mRNA band was also observed (lane 5). More importantly, the rat cDNA clone was capable of cross-hybridizing with phenylalanine hydroxylase mRNAs from baboon and human (lanes 6 and 7), suggesting that the corresponding human genes can be cloned using the rat phenylalanine hydroxylase cDNA clone as a probe.

Preliminary Characterization of Rat Phenylalanine Hydroxylase cDNA Clones

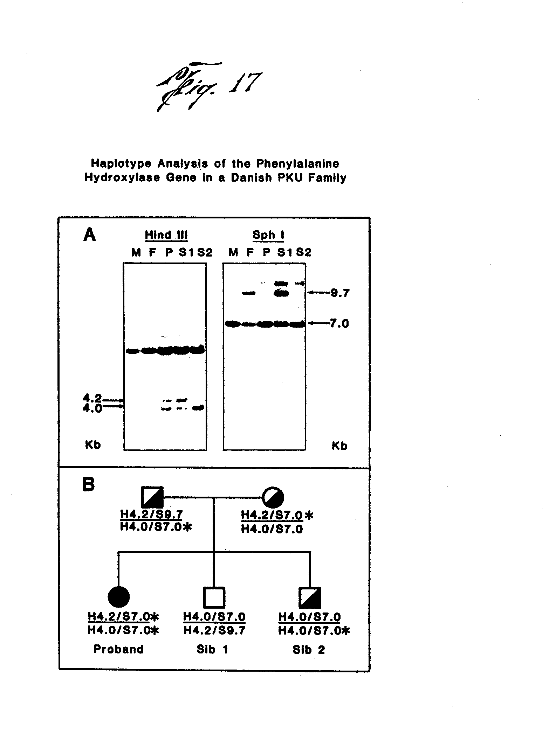

Independently identified rat phenylalanine hydroxylase cDNA clones were analysed by restriction mapping. Partial restriction maps of the two longest overlapping rat phenylalanine hydroxylase cDNA clones, prPH91 and prPH98, are shown in Fig. 11.

Cloning of the Human Phenylalanine Hydroxylase cDNA

Total cellular RNA was extracted using phenol-SDS from a pathologic human liver specimen. Poly (A)-continuing RNA was prepared by oligo (dT)-cellulose column chromatography. The RNA preparation was used for cDNA synthesis under conditions that favored full-length cDNA production (Monahan et al, 1976, Biochemistry 15, 223-233). The cDNA preparation was sedimented through an alkaline sucrose gradient (Monahan et al, 1976, Biochemistry 15, 223-233) and only fractions containing cDNA species of 1,000 nucleotides or greater were pooled. The single-stranded cDNA was subsequently made double-stranded with reverse transcriptase (Monahan et al, 1976, J. Biol. Chem. 251, 7355-7362). After S1 nuclease treatment, tracts of polydC were added onto the 3' termini of the DNA molecules using terminal transferase (Chandra et al, 1981, Biochem. Biophys. Res. Commun. 103, 751-758). The enzymatically synthesized cDNA was reannealed with Pst I-linearized pBR322 DNA that had been tailed with poly dG and the DNA was used for transformation of E. coli RR1 as described above for the rat cDNA clones (Chandra et al, 1981, Biochem. Biophys. Res. Commun. 103, 751-758). These experiments were performed according to the guidelines for recombinant DNA research from the National Institute of Health. Bacterial transformants were selected for resistance to tetracycline and approximately 40,000 individual recombinants containing human liver cDNA sequences were obtained. Analysis of inserted DNA lengths in 20 randomly selected colonies by mini-lysis (Birnboim, H.C. and Doly, J., 1979, Nucleic Acids Res. 7, 1513-1523) and agarose gel electrophoresis has indicated that greater than 95% of the transformants were indeed recombinants, and the average length of human DNA in these recombinants was approximately 1.2 kilobase pairs.

Using the previously cloned rat phenylalanine hydroxylase cDNA clone (prPH98) as a specific hybridization probe, the human liver cDNA library comprising of 40,000 independent transformants was screened. Recombinant plasmid DNAs were isolated from the bacterial colonies that yielded strong hybridization signals. The DNA samples were analyzed by Southern hybridization after digestion with the restriction enzyme HhaI which cleaved the plasmid vector DNA into small fragments, but did not cut the inserted human DNA in any of the clones. As positive controls of the experiment, the two previously cloned rat phenylalanine hydroxylase cDNA recombinants, prPH91 and prPH98, were included and strong hybridization signals were obtained (Fig. 12, lanes 1 and 2). The specificity of the hybridization probe has been demonstrated by the lack of hybridization with the vector DNA pBR322 (Fig. 12, lane 3) and a previously cloned human alpha 1-antitrypsin cDNA clone (Fig. 12, lane 4). Specific hybridization signals were obtained from the phenylalanine hydroxylase cDNA clones identified from the human liver cDNA library (Fig. 12, lanes 5-10). The lengths of the inserted human DNA in these recombinant plasmids ranged from 0.3-1.4 kb. Partial DNA sequence analysis has indicated that extensive sequence homology between the rat and human phenylalanine hydroxylase cDNA clones. These clones were characterized by restriction mapping, and the structure of the 2 recombinants containing largest overlapping inserted human DNA fragments, phPH72 and phPH73, are shown in Fig. 13.

### EXAMPLE 1

Genomic DNAs were isolated from 2 PKU cell lines obtained from the human mutant cell depository and from 2 normal individuals. The DNA preparations were disgested with a variety of restriction enzymes followed by Southern hybridization using the two human phenylalanine hydroxylase cDNA clones as hybridization probes. Identical hybridization signals were obtained from all 4 DNA preparations after digestion with multiple enzymes, indicating that the phenylalanine hydroxylase gene is not only still present in the genome of cells derived from PKU patients, but the overall organization of the gene also remains unchanged. Since the cDNA probes were only 1.4-kb in length and the human phenylalanine hydroxylase mRNA is comprised of approximately 2,500 nucleotides, it is still possible that there could be a deletion or rearrangement in regions of the phenylalanine hydroxylase gene that are not represented in the cDNA clones. Consequently, it could only be concluded that classical phenylketonuria, at least in these 2 cases, is not caused by deletion of the entire pheylalanine hydroxylase gene. This observation has subsequently been extended to include several additional phenylketonuric individuals analyzed in the following sections.

EXAMPLE 2

It has recently been established that there exists a high number of nucleotide substitutions in the genome of different individuals. These substitutions are usually without phenotypic expression and can only be detected by restriction enzymes that cleave DNA at the respective recognition sequences. These random restriction site polymorphisms result in a difference of the DNA fragment length, which can be readily detected by agarose gel electrophoresis followed by Southern hybridization using specific hybridization probes. Restriction fragment length polymorphism has since become a powerful tool for molecular analysis of human genetic disorders. In order to apply this technology to analyze classical phenylketonuria, it is imperative to identify the existence of polymorphic restriction sites in the phenylalanine hydroxylase locus. Genomic DNAs isolated from 20 random normal Caucasians in the Houston metropolitan area were digested by a battery of restriction enzymes and the resulting DNA fragments were analyzed by agarose gel electrophoresis followed by Southern hybridization. Although most restriction enzymes yielded identical patterns between these individuals, 3 enzymes that yielded polymorphic patterns in the phenylalanine hydroxylase locus were identified. The enzyme MspI generated a single hybridization band at 23-kb in some individuals and at 19-kb in others (Fig. 14A, lanes 1 and 2) indicating the existence of 2 genotypic alleles. Indeed, heterozygotes containing both bands have also been detected (Figure 14A, lane 3). The frequencies of the alleles associated were the 23-kb and 19-kb bands are 0.444 and 0.556, respectively.

The existence of a second allelic system in the human phenylalanine hydroxylase locus has been identified by

-23-

the enzyme SphI, which cleaved the DNAs into either a 7.0-kb or a 9.7-kb fragment in different individuals (Fig. 14B, lanes 1 and 2). The presence of both bands in heterozygotes has also been detected (Fig. 1B, lane 3). The 11.0-kb DNA fragment is present in every individual and is therefore non-allelic. In this case, the frequencies for the 9.7-kb and 7.0-kb alleles are 0.059 and 0.941, respectively.

The enzyme HindIII has revealed a 3 allelic system. Most individuals are either homozygous or heterozygous for the 4.2-kb and 4.0-kb bands (Fig. 1C, 1, 2 and 4), the frequencies of which are 0.706 and 0.235, respectively. A rarer 4.4-kb band has also been detected in some individuals, with a frequency of 0.059 (Fig. 1C, lane 3). The existence of these allelic systems permits the analysis of classical phenylketonuria by restriction fragment length polymorphism.

EXAMPLE 3

Seven Danish families with histories of classical phenylketonuria in 1 or 2 children were analyzed by restriction fragment length polymorphism. In one such family, both parents are heterozygous for the 23 kb and 19 kb alleles as revealed by the enzyme MspI (Fig. 15A, lanes 1 and 2). Since both parents are obligatory heterozygotes for the PKU trait, each must contain a mutant phenylalanine hydroxylase gene which could be associated with either allele. The proband is homozygous for the 19 kb allele (Fig. 15A, lane 3), indicating that the mutant genes of both parents reside in the 19 kb alleles. An unaffected sibling in this family is homozygous for the 23 kb allele (Fig. 15A, lane 4), indicating that this person has inherited both non-phenylketonuric genes from the parents and should therefore be free of the phenylketonuric trait. A diagram illustrating the Mandelian segregation of the 2 alleles in this family is also shown (Fig. 15A). These results indicate that prenatal diagnosis for classical phenylketonuria will be possible in future pregnancies of this family by analysis of DNA isolated from

0126544

-24-

amniotic cells of fetuses using the MspI restriction fragment length polymorphism test. A fetus homozygous for the 19 kb allele will be phenylketonuric, one that is heterozygous for the 2 MspI alleles will be a trait carrier and one that is homozygous for the 23 kb allele will be free of the PKU trait.

The PKU alleles could also be identified by SphI and HindIII polymorphism in some families. Both parents are heterozygous for the 9.7 and 7.0 kb alleles in one such family as revealed by SphI (Fig. 15B, lanes 1 and 2). The proband in this family is homozygous for the 9.7 kb allele (Fig. 15B, lane 3), while an unaffected sibling is homozygous for the 7.0 kb allele and is therefore not a trait carrier (Fig. 15B, lane 4). In another family, both parents' DNA are heterozygous for the 4.2 kb and 4.0 kb alleles after digestion by the enzyme HindIII (Fig. 15C, lanes 1 and 2). The proband is homozygous for the 4.0 kb allele (Fig. 15C, lane 3). An unaffected sibling in this family is heterozygous for the 2 alleles (Fig. 15C, lane 4) and is thus a trait carrier. In both of these families, it is apparent that carrier detection and prenatal diagnosis will be possible by simple restriction fragment length polymorphism analysis of fetal DNA using the respective enzymes.

EXAMPLE 4

The existence of 3 allelic systems as revealed by HindIII digestion of genomic DNA has made this enzyme particularly useful in analysis of classical phenylketonuria. In one of the Danish families analysed, the father was heterozygous for the 4.4 kb and 4.2 kb alleles (Fig. 16A, lane 1) while the mother was heterozygous for the 4.2 kb and 4.0 kb alleles (Fig. 16A, lane 2). The proband in this family was heterozygous for the 4.4 kb and 4.2 kb alleles (Fig. 4A, lane 3). This individual must have inherited the 4.4 kb allele from the father and the 4.2 kb allele from the mother which must be the mutant alleles. An unaffected sibling in this family was heterozygous for 4.2 kb and 4.0 kb alleles (Fig. 16A, lane 4), who must have inherited the 4.0 allele from the mother and the 4.2 kb allele from the father. Since both parents are obligatory heterozygous for the PKU trait, these 2 alleles in both parents must not be phenylketonuric and the sibling must therefore be free of the PKU trait.

### EXAMPLE 5

There were 2 probands in one of the Danish families. This family is particularly important in that the segregation of the PKU alleles in both probands must be concordant if the genetic disorder is indeed caused by a mutation in the phenylalanine hydroxylase gene itself but not through a trans-regulatory mechanism. HindIII polymorphism has been detected in both parents who are heterozygous for the 4.2 kb and 4.0 kb alleles (Fig. 16B, lanes 1 and 2). One of the probands is homozygous for the 4.2 kb allele (Fig. 16B, lane 3), indicating that the PKU genes in this family are associated with the 4.2 kb alleles. A second proband is also homozygous for the 4.2 kb allele (Fig. 16B, lane 4). Since the probability of a random occurance of identical genotypes in the 2 probands is only 1 out of 4, the results indicate that the segregation of the alleles and disease state in this family are indeed concordant. This interpretation is further strengthened by the observation that 2 unaffected siblings in the same family have inherited different alleles from the parents. One sibling was homozygous for the 4.0 kb alleles (Fig. 16B, lane 5) who was free of the trait, another sibling was heterozygous for the 4.2 kb and 4.0 kb alleles (Fig. 16B, lane 6) who must be a trait carrier. Concordant segregation between the mutant alleles and disease state has been observed in another Danish PKU family with 2 probands. Taken together with the segregation discordance of the proband and unaffected siblings in all families analyzed, the data strongly suggest that classical phenylketonuria is

-26-

indeed caused by underlying mutations in the phenylalanine hydroxylase gene itself, and restriction fragment length polymorphism in the phenylalanine hydroxylase locus as revealed by the human phenylalanine hydroxylase cDNA clones can indeed be used for prenatal diagnosis and carrier detection of classical PKU in certain families.

## EXAMPLE 6

Haplotype identification of genetic alleles in man is an extremely powerful tool for analysis of hyman genetic disorders. The exploitation of this technology in the analysis of various forms of Thalmassemias has been well documented recently. The application of haplotype analysis of the phenylalanine hydroxylase gene in one of the Danish families is shown in Fig. 17. In this family, although both parents were heterozygous for the 4.2 kb and 4.0 kb HindIII alleles (Panel A, lanes M and F), the proband is also heterozygous for the 2 alleles (Panel A, lane P). Consequently the mutant genes cannot be associated with either allele in both parents by the analysis alone. SphI digestion of the parents' DNA however, has shown that the mother was homozygous for the 7.0 kb allele while the father was heterozygous for the 9.7 kb and 7.0 kb alleles (Panel A, lanes M and F). Since the proband is homozygous for the 7.0 kb allele (Panel A, lane P), the father's PKU gene must be associated with the 7.0 kb allele. This PKU gene has been transmitted into one of the unaffected siblings who was also homozygous for the 4.0 kb HindIII allele (Panel A, lane S2), the PKU gene he inherited from the father must therefore have a haplotype of H4.0/S7.0, where H and S represent HindIII and SphI, respectively. The haplotype of the father's non-PKU gene must therefore be H4.2/S9.7. Once the haplotypes of the father's phenylalanine hydroxylase genes are defined, those of the mother's genes can be readily deduced. Since the proband is heterozygous for the 4.2 kb and 4.0 kb HindIII alleles (Panel A, lane P), she must have inherited the 4.2 kb HindIII allele from the mother, which must be the PKU gene. Since the mother is homozygous for the 7.0 kb SphI allele, her PKU gene has a haplotype of H4.2/S7.0 and her non-PKU gene has a haplotype of H4.0/S7.0. Once the haplotypes of all 4 phenylalanine hydroxylase genes in this family are established, there can be a total of four possible combinations of the phenylalanine hydroxylase gene's haplotypes in all children of this family, three of which are represented in the three existing children (Fig. 17b). It becomes apparent that the other unaffected sibling was homozygous for the 4.0 kb HindIII alleles and heterozygous for the 9.7 kb and 7.0 kb SphI alleles (Panel A, lane S1). This individual has inherited the non-PKU gene of the H4.2/S9.7 haplotype from the father and the non-PKU gene of the H4.0/S7.0 haplotype from the mother and was consequently free of the PKU trait.

### EXAMPLE 7

In one of the families, both parents were homozygotes when analyzed by MspI and SphI polymorphism. Analysis of this family with HindIII has indicated that the father was heterozygous for the 4.2 kb and 4.0 kb alleles, the mother was homozygous for the 4.2 kb allele and the proband was also homozygous for the 4.2 kb allele (data not shown). An unaffected sibling was heterozygous for the two alleles and in this case, one cannot determine whether this individual was a trait carrier or free of the PKU trait by polymorphism analysis. However, the possibility of this individual to be of the PKU phenotype can be excluded on the basis that the father's non-PKU gene has been transmitted into this individual. One the other hand, if the fetal DNA has a homozygous 4.2 kb genotype in a future pregnancy, one cannot determine whether the fetus will be a trait carrier or a phenylketonuric individual and exclusion cannot be affected. The probability of possible exclusion of PKU homozygotes in this family is 50%, which will also be true for all families with a homozygous and a heterozygous parent.

-28-

## EXAMPLE 8

Since prenatal diagnosis and heterozygote detection by restriction fragment length polymorphism depends strictly on at least one parent being a haplotype heterozygote, it is important to establish the percent heterozygosity in any given individual within the general population. Since there are 3 <u>Hind</u>III alleles, 2 <u>Msp</u>I alleles and 2 <u>Sph</u>I alleles, there are a total of 12 theoretical genotypic alleles of the human phenylalanine hydroxylase gene by the present analysis, each with a characteristic haplotype (Table 1). The frequency of each of the haplotype will be the product of the individual allelic frequencies as revealed by the 3 restriction enzymes. The probability of an individual to be homozygous for any one of the haplotypes would be the square of the haplotype frequency, and the sum of the square of all 12 haplotype frequencies would represent the frequency of homozygous individuals in the population. The rest of the individuals must be heterozygous for any 2 of the haplotypes, and percent heterozygosity should be the percent homozygosity subtracted from 100% and is equal to 75% in this case (Table 1).

Table 1. Haplotypes and Percent Heterozygosity of

Human Phenylalanine Hydroxylase Gene

| Haplotype Number | HindIII | | MspI | | SphI | | Specific Haplotypes | Haplotype Frequency* | Percent Heterozygosity |
|---|---|---|---|---|---|---|---|---|---|
| | Allele | Frequency | Allele | Frequency | Allele | Frequency | | | |
| 1 | 4.4 | 0.059 | 23 | 0.444 | 9.7 | 0.059 | H4.4/M23/S9.7 | 0.0015 | |
| 2 | 4.4 | 0.059 | 23 | 0.444 | 7.0 | 0.941 | H4.4/M23/S7.0 | 0.0246 | |
| 3 | 4.4 | 0.059 | 19 | 0.556 | 9.7 | 0.059 | H4.4/M19/S9.7 | 0.0019 | |
| 4 | 4.4 | 0.059 | 19 | 0.556 | 7.0 | 0.941 | H4.4/M19/S7.0 | 0.0308 | |
| 5 | 4.2 | 0.706 | 23 | 0.444 | 9.7 | 0.059 | H4.2/M23/S9.7 | 0.0185 | |
| 6 | 4.2 | 0.706 | 23 | 0.444 | 7.0 | 0.941 | H4.2/M23/S7.0 | 0.2949 | 75.0% |
| 7 | 4.2 | 0.706 | 19 | 0.556 | 9.7 | 0.059 | H4.2/M19/S9.7 | 0.0232 | |
| 8 | 4.2 | 0.706 | 19 | 0.556 | 7.0 | 0.941 | H4.2/M19/S7.0 | 0.3694 | |
| 9 | 4.0 | 0.235 | 23 | 0.444 | 9.7 | 0.059 | H4.0/M23/S9.7 | 0.0062 | |
| 10 | 4.0 | 0.235 | 23 | 0.444 | 7.0 | 0.941 | H4.0/M23/S7.0 | 0.0982 | |
| 11 | 4.0 | 0.235 | 19 | 0.556 | 9.7 | 0.059 | H4.0/M19/S9.7 | 0.0078 | |
| 12 | 4.0 | 0.235 | 19 | 0.556 | 7.0 | 0.941 | H4.0/M19/S7.0 | 0.1229 | |

*In the absence of data from a large number of families, which is a pre-requisite to establish accurate haplotype frequencies in the population, these figures are derived from theoretical calculations based on the frequencies of the individual alleles. The possibility of linkage disequilibrium between the polymorphisms has not been taken into consideration in these calculations.

## EXAMPLE 9

In the general population, the probabilities of mating events between 2 heterozygotes, 2 homozygotes, and between a heterozygote and a homozygote are governed by the binomial expansion $(75\% + 25\%)^2$, where 75% and 25% are percent heterozygosity and percent homozygosity, respectively. Thus the probability of 2 heterozygotes mating would be $(75\%)^2$ or 56.25%. In these events, if the haplotypes of the PKU genes in the parents can be identified by comparison with the proband's haplotypes, complete prenatal diagnosis can be expected by analysis of fetal DNA through amniocentesis. In the event that a pregnancy has resulted from mating between 2 homozygotes (6.25%), there can be no diagnosis and obviously amniocentesis should not be performed. Finally, if a heterozygote mates with a homozygote, only 50% of the resulting offsprings can be excluded from having PKU phenotypes. Since the mating event has a probability of 37.5%, the overall probability of excluding recessive homozygotes will be 18.75% (Table 2).

Table 2.  Probability of Complete Diagnosis or Exclusion of PKU Homozygotes

| Parents | Heterozygote (75%) X Heterozygote (75%) | Heterozygote (75%) X Homozygote (25%) | Homozygote (25%) X Homozygote (25%) |
|---|---|---|---|
| Probability[a] | 56.25% | 37.50% | 6.25% |
| Amniocentesis | + | + | − |
| Diagnosis | + | NA[b] | − |
| Exclusion | + | + − | − |
|  |  | (18.75%)   (18.75%) |  |

a   Probability of matings between heterozygotes and homozygotes is calculated according to the Bionomial expansion equation:

$$(75\% + 25\%)^2 = (75\%)^2 + 2 \times (75\%)(25\%) + (25\%)^2 = 56.25\% + 37.50\% + 6.25\%$$

b   NA:  not applicable

Thus, a cloned rat phenylalanine hydroxylase cDNA clone has been used to identify the corresponding human cDNA clones in a human liver cDNA library by cross hybridization. The human phenylalanine hydroxylase cDNA clones have been employed as a hybridization probe to analyze classical phenylketonuria. Conclusive evidence demonstrating the presence of the phenylalanine hydroxylase gene in cellular DNA of the PKU phenotype has been obtained, suggesting that classical phenylketonuria is not caused by a deletion of the entire phenylalanine hydroxylase gene.

Since the initial observation by Kan and Dozy (1978, Proc. Natl. Acad. Sci. USA $\underline{75}$, 5631) that there is a HpaI restriction site polymorphism at the 3' flanking region of the human beta-globin gene which is associated and can be used for diagnosis of sickle-cell anemia, restriction fragment length polymorphism has developed into an extremely powerful tool to analyze human genetic disorders. It has now been established that there are over 10 enzymes that would yield polymorphic patterns from the 60 kb human beta-globin locus, and the application of these enzyme polymorphisms for prenatal diagnosis of various kinds of beta° and beta+ thalassemia by linkage analysis has been well documented. Using the cloned human phenylalanine hydroxylase cDNA probe, 3 restriction enzymes have been identified that yielded polymorphic patterns from the phenylalanine hydroxylase locus, resulting in a total of 12 theoretical haplotypes of the gene. Based on a rather small sample size (20 random individuals) and not taking into consideration the possibility of linkage dis-equilibrium between these polymorphic sites, it has been calculated that the percent heterozygosity within the general Caucasian population is 75%. This high level of heterozygosity would suggest that prenatal diagnosis can be performed for 56.25% of the random PKU families and exclusion of recessive homozygotes can be achieved in an additional 18.75% of the families. These probabilities however, are optimal estimates in that the

0126544

haplotypes of all 4 phenylalanine hydroxylase genes in a particular family may not be identifiable by comparison with the proband alone. A typical example is provided in the family shown in Fig. 4. Without the availability of the informative sibling S2, complete diagnosis would not have been possible. Thus the real probability for diagnosis and exclusion could depend also on the availability of additional siblings or grandparents in order to establish phase for the haplotypes of the gene. Consequently, under ideal conditions, genetic services can be provided for up to a total of 75% of the random PKU families in the general Caucasian population. These numbers however, could only improve in the future with the identification of additional alleles by restriction polymorphism when more DNA in the human phenylalanine hydroxylase gene become cloned and used as hybridization probes.

The present invention, therefore, is well suited and adapted to attain the objects and ends and has the advantages mentioned as well as others inherent therein.

-1-

## CLAIMS

1. A method of synthesizing cDNA clones from phenylalanine hydroxylase mRNA from liver tissue comprising,

isolating and purifying phenylalanine hydroxylase from the liver tissue,

obtaining and isolating antibodies to the purified phenylalanine hydroxylase,

obtaining polysome enriched phenylalanine hydroxylase mRNA by immunoprecipitation of liver polysomes using the antibodies, and

enzymatically synthesizing recombinant cDNA clones from the polysome enriched phenylalanine hydroxylase mRNA.

2. The method of claim 1 where the liver tissue is human liver tissue.

3. The method of claim 1 where the liver tissue is animal liver tissue.

4. The method of claim 1 where the liver tissue is rat liver tissue.

5. Phenylalanine hydroxylase cDNA clone.

6. Human phenylalanine hydroxylase cDNA clone.

7. Animal phenylalanine hydroxylase cDNA clone.

8. Rat liver phenylalanine hydroxylase cDNA clone.

9. A labelled phenylalanine hydroxylase cDNA clone.

10. A labelled hyman phenylalanine hydroxylase cDNA clone.

11. A labelled animal phenylalanine hydroxylase cDNA clone.

12. A labelled rat phenylalanine hydroxylase cDNA clone.

13. A method of diagnosing phenylketonuria prenatally comprising,

gene mapping utilizing human phenylalanine hydroxylase cDNA clones as probes.

14. A method of diagnosing phenylketonuria prenatally comprising,

cleaving fetal DNA with enzymes,

cleaving available parents' DNA with said enzymes,

cleaving DNA from available unaffected siblings with said enzymes,

cleaving DNA from available previously affected siblings with said enzymes, and

comparing the polymorphism of the cleaved DNAs.

15. A method of constructing a human cDNA library from which human phenylalanine hydroxylase cDNA clones can be identified and isolated using a corresponding animal phenylalanine hydroxylase cDNA clone by cross-hybridization comprising,

synthesizing cDNA clones from the total human liver mRNA,

inserting the synthesized cDNA clone into plasmid DNA,

introducing the resulting recombinant plasmid DNA into a suitable bacterium to construct the human liver cDNA library,

hybridizing labelled animal cDNA clones with the human livery library cDNA clones, and

determining positive signals.

16. E. Coli RR1 (prPH91) having the deposit accession number ATCC 39329.

17. E. Coli RR1 (prPH98) having the deposit accession number ATCC 39330.

18. E. Coli RR1 (phPH72) having the deposit accession number ATCC 39331.

19. E. Coli RR1 (phPH73) having the deposit accession number ATCC 39332.

Fig. 1

*Classical Phenylketonuria: deficiency of phenylalanine hydroxylase
*Hyperphenylalaninemia: reduced levels of phenylalanine hydroxylase
*Atypical Phenylketonuria: deficiency of dihydropteridndine reductase

Fig. 2

*Fig. 3*

Tissue
| Homogenization

—AA —AAA —AAAA Polysomes

| Specific IgG (Y)

—AA —AAA —AAAA

| 1. S. aureus cells
| 2. Centrifugation

—AAA

S. aureus

| 1. Extract with Phenol
| 2. Oligo dT—Cellulose

~AAA   Enriched mRNA

*Fig. 4*

Fig. 5

Reticulocyte lysate + mRNA

[35S] methionine

A    AA    AAA

1. Specific IgG
2. Centrifugation

Antibody-Antigen
pellet

1. Disruption of protein
   complexes by SDS
2. Polyacrylamide gel
   electrophoresis
3. Fluorography of gel

Total Protein
Products

Specific Protein
Product

Fig. 8

(Recombinant Plasmid DNA)

Digestion with
restriction enzyme

1. Thermal denaturation
2. Coupling to DBM-cellulose

AA  AA
total cellular
mRNA

Hybridization

AA  AA
AA  AA

1. Extensive wash
2. Thermal denaturation

AA    (Hybrid-Selected mRNA)

*Fig. 6*

0126544

Fig. 7

Fig. 9

A 1 2 3 4 5 6 7     M.Wt.

92,500
69,000
46,000
30,000

B     M.Wt.

92,500
69,000
46,000
30,000

Fig. 10

1   2   3   4   5   6   7   8

28S
18S

Fig. 11

Fig. 13

Fig. 12

Fig. 14

Fig. 16

A

1 2 3 4

5.6
5.2
4.4
4.3
4.0
2.7
Kb

H4.2*/H4.0
H4.2/H4.0
H4.4*/H4.2
H4.4*/H4.2*

B

1 2 3 4 5 6

5.6
5.2
4.2
4.0
2.7
Kb

H4.2*/H4.0
H4.2*/H4.0
H4.0/H4.0
H4.2*/H4.2*
H4.2*/H4.2*
H4.2*/H4.0

Fig. 15

A

1 2 3 4

23.0
19.0
Kb

M23/M19*
M23/M23
M23/M19*
M19/M19*

B

1 2 3 4

11.0
9.7
7.0
Kb

S9.7/S7.0
S7.0/S7.0
S9.7/S7.0
S9.7/S9.7*

C

1 2 3 4

5.6
5.2
4.2
4.0
2.7
Kb

H4.2/H4.0*
H4.2/H4.0*
H4.2/H4.0*
H4.0/H4.0*

*Fig. 17*

**Haplotype Analysis of the Phenylalanine
Hydroxylase Gene in a Danish PKU Family**